# EUROPEAN PATENT APPLICATION

(11) **EP 4 693 305 A1**
(43) Date of publication of application: **11.02.2026**
(21) Application number: 24778579.3
(22) Date of filing: 19.01.2024
(51) Int. Cl.: G16C 20/30, G16C 20/80

(54) **INFORMATION PROCESSING DEVICE, INFORMATION PROCESSING METHOD, AND PROGRAM**

(30) Priority: 31.03.2023 JP 2023059424
(71) Applicant: FUJIFILM Corporation, Tokyo 106-8620 (JP)
(72) Inventor: TATESHITA, Masakazu, Ashigarakami-gun, Kanagawa 258-8577 (JP); YARIMIZU, Hirokazu, Ashigarakami-gun, Kanagawa 258-8577 (JP); HIKIDA, Yasushi, Ashigarakami-gun, Kanagawa 258-8577 (JP); MURAKAMI, Ryoichi, Ashigarakami-gun, Kanagawa 258-8577 (JP); SUGIYAMA, Satoshi, Ashigarakami-gun, Kanagawa 258-8577 (JP)
(74) Representative: HGF
(86) International application number: PCT/JP2024/001438
(87) International publication number: WO 2024/202433

(57) **Abstract**

An information processing apparatus includes a processor, in which the processor is configured to output, to a display unit, an inquiry image in which an evaluation result of a toxicity evaluation of a compound is capable of being displayed, the inquiry image including an operation unit that receives an operation of transmitting an inquiry notification regarding the evaluation result to a destination designated in advance, and in a case where the operation unit is operated, execute control of transmitting, to the destination, the inquiry notification including information that is shareable with the destination and that includes at least a part of the evaluation result.

## Description

### BACKGROUND OF THE INVENTION

### 1. Field of the Invention

The technology of the present disclosure relates to an information processing apparatus, an information processing method, and a program.

### 2. Description of the Related Art

In recent years, in the field of handling chemical substances, a toxicity evaluation using a computer-based in silico method has become common. The toxicity evaluation of the chemical substance requires, in addition to simply obtaining the toxicity evaluation, various examinations from the viewpoint of causes of toxicity manifestation or a tendency of a compound that is likely to manifest toxicity.

JP2004-174331A discloses an automatic synthesis and evaluation system of a compound, including: synthesis units 101 and 102 that synthesize a plurality of compounds by a liquid phase method or a solid phase method in accordance with synthesis conditions for forming a combinatorial chemical library; evaluation units 106 to 109 that evaluate chemical, physical, or biological properties of the compound; and a main control unit 121 that sets the synthesis condition for the synthesis unit, performs calculation processing on evaluation results of the properties obtained from the evaluation unit for each compound obtained under the synthesis condition, and has a memory that registers a database for forming the combinatorial chemical library.

JP2019-20791A discloses a method of predicting toxicity of a compound, including: (1) a step of receiving structural information of a test compound input by a user; (2) a step of generating a three-dimensional molecular structure with an optimized structure, based on the received structural information; (3) a step of using the three-dimensional molecular structure to generate values of one or more molecular descriptors including at least one molecular descriptor selected from the group consisting of a three-dimensional molecular descriptor, a four-dimensional molecular descriptor, and a quantum chemical molecular descriptor; (4) a step of calculating a probability of presence or absence of toxicity of the test compound by a toxicity prediction model using the values of the molecular descriptors, where the sum of the probability of presence of toxicity and the probability of absence of toxicity is 100%; and (5) a step of outputting the calculated probability.

JP2020-25471A discloses a toxicity learning device comprising: an input unit that inputs expression data of a sample exposed to a compound and expression data of a control; a comparison unit that compares expression data of the sample and the control for each predetermined gene; an encoding unit that encodes expression data of the gene based on a difference in expression data; a label assignment unit that assigns a label of toxicity of the compound to the encoded expression data; and a model learning unit that trains a model for predicting the toxicity of the compound from the expression data of the gene using training data to which the label is assigned.

### SUMMARY OF THE INVENTION

In the technologies disclosed in JP2004-174331A, JP2019-20791A, and JP2020-25471A, although the method of evaluating toxicity is taken into consideration, no consideration has been given to making the evaluation result easier for a user to interpret. In the toxicity evaluation of the compound, in a case where the user wishes to further examine the findings, for example, an inquiry regarding the evaluation result may be made to an external expert or a specialized organization. In this case, the user needs to inquire about the evaluation result from an external expert or the like using a method other than software used for the toxicity evaluation, and extra effort is required to share the evaluation result. Therefore, there is room for improvement in sharing the evaluation result of the toxicity of the compound and supporting the evaluation of the toxicity of the compound.

One embodiment according to the technology of the present disclosure provides an information processing apparatus, an information processing method, and a program capable of sharing an evaluation result of toxicity of a compound and supporting a user in evaluating the toxicity of the compound.

A first aspect according to the technology of the present disclosure is an information processing apparatus comprising: a processor, in which the processor is configured to output, to a display unit, an inquiry image in which an evaluation result of a toxicity evaluation of a compound is capable of being displayed, the inquiry image including an operation unit that receives an operation of transmitting an inquiry notification regarding the evaluation result to a destination designated in advance, and in a case where the operation unit is operated, execute control of transmitting, to the destination, the inquiry notification including information that is shareable with the destination and that includes at least a part of the evaluation result.

A second aspect according to the technology of the present disclosure is the information processing apparatus according to the first aspect, in which the inquiry image is displayed by transitioning from a list image showing a result of performing the toxicity evaluation on each of compounds included in a plurality of compound groups as toxicity evaluation targets, in a case where at least one of the compounds is selected from the list image.

A third aspect according to the technology of the present disclosure is the information processing apparatus according to the second aspect, in which the list image is capable of being sorted and/or filtered out according to a condition designated by a user.

A fourth aspect according to the technology of the present disclosure is the information processing apparatus according to the first aspect, in which the evaluation result includes a toxicity evaluation result for each toxicity item.

A fifth aspect according to the technology of the present disclosure is the information processing apparatus according to the fourth aspect, in which the toxicity evaluation result includes a determination flow indicating a toxicity determination procedure, which is used in the toxicity evaluation, has at least one determination step, and has a path that branches according to a determination result of the determination step, and in which, among a plurality of paths, a path that leads to the toxicity evaluation result of the compound is shown in a distinguishable manner from the other paths.

A sixth aspect according to the technology of the present disclosure is the information processing apparatus according to the fifth aspect, in which the toxicity evaluation result includes an explanation of a determination content for each determination step included in the determination flow.

A seventh aspect according to the technology of the present disclosure is the information processing apparatus according to the first aspect, in which the inquiry image includes an input region in which an inquiry content included in the inquiry notification is capable of being input.

An eighth aspect according to the technology of the present disclosure is the information processing apparatus according to the seventh aspect, in which the input region is a text input region in which a text indicating the inquiry content is capable of being input.

A ninth aspect according to the technology of the present disclosure is the information processing apparatus according to the second aspect, in which, in a case where a plurality of compounds are selected in the list image, the information that is shareable with the destination includes at least a part of the evaluation result of each of the plurality of compounds.

A tenth aspect according to the technology of the present disclosure is an information processing method comprising: outputting, to a display unit, an inquiry image in which an evaluation result of a toxicity evaluation of a compound is capable of being displayed, the inquiry image including an operation unit that performs an operation of transmitting an inquiry notification regarding the evaluation result to a destination designated in advance; and executing, in a case where the operation unit is operated, control of transmitting, to the destination, the inquiry notification including information that is shareable with the destination and that includes at least a part of the evaluation result.

An eleventh aspect according to the technology of the present disclosure is a program for causing a computer to execute a process comprising: outputting, to a display unit, an inquiry image in which an evaluation result of a toxicity evaluation of a compound is capable of being displayed, the inquiry image including an operation unit that performs an operation of transmitting an inquiry notification regarding the evaluation result to a destination designated in advance; and executing, in a case where the operation unit is operated, control of transmitting, to the destination, the inquiry notification including information that is shareable with the destination and that includes at least a part of the evaluation result.

The technology of the present disclosure provides an information processing apparatus, an information processing method, and a program capable of sharing an evaluation result of toxicity of a compound and supporting a user in evaluating the toxicity.

### BRIEF DESCRIPTION OF THE DRAWINGS

FIG. 1 is a conceptual diagram showing a schematic configuration example of an information processing system.
FIG. 2 is a block diagram showing an example of a hardware configuration of an electrical system of a server.
FIG. 3 is a conceptual diagram showing an example of a usage aspect of the information processing system.
FIG. 4 is a functional block diagram showing an example of main functions of a processor of the server.
FIG. 5 is a conceptual diagram showing an example of processing contents of a toxicity evaluation unit.
FIG. 6 is a conceptual diagram showing an example of processing contents of an image generation unit.
FIG. 7 is a conceptual diagram showing an example of an aspect in which a part of a display image is displayed on a display device.
FIG. 8 is a conceptual diagram showing an example of the display image.
FIG. 9 is a conceptual diagram showing an example of the display image.
FIG. 10 is a conceptual diagram showing an example of an aspect in which an inquiry notification is transmitted.
FIG. 11 is a flowchart showing an example of a flow of server control processing.
FIG. 12 is a conceptual diagram showing an example of an aspect in which an inquiry notification is transmitted.
FIG. 13 is a conceptual diagram showing an example of an aspect in which a part of the display image is displayed on the display device.
FIG. 14 is a conceptual diagram showing an example of an aspect in which an inquiry notification is transmitted.
FIG. 15 is a block diagram showing an example of a hardware configuration of an electrical system of a client terminal.
FIG. 16 is a functional block diagram showing an example of main functions of a processor of the client terminal.
FIG. 17 is a functional block diagram showing an example of main functions of the processor of the client terminal.

### DESCRIPTION OF THE PREFERRED EMBODIMENTS

An example of embodiments of an information processing apparatus, an information processing method, and a program according to the technology of the present disclosure will be described with reference to the accompanying drawings.

### [First Embodiment]

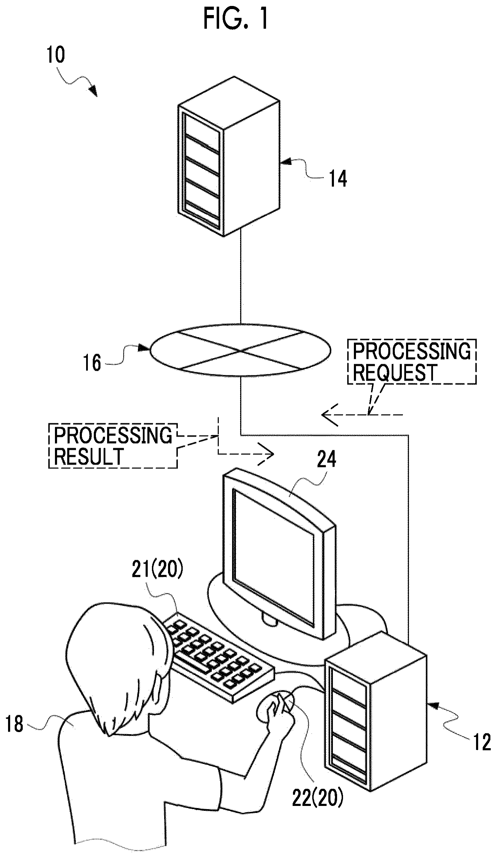

As shown in FIG. 1 as an example, an information processing system 10 comprises a client terminal 12 and a server 14. In the information processing system 10, the client terminal 12 and the server 14 are communicably connected to each other via a network 16.

The information processing system 10 is used, for example, for evaluation of characteristics of a chemical substance (for example, evaluation of the presence or absence of toxicity to a human body). A user 18 (for example, a researcher) can use the information processing system 10 to predict and evaluate experimental results using computer simulations (that is, an evaluation using in silico method). For example, for a newly developed or under-development chemical substance, it is possible to obtain an evaluation result of toxicity without performing a toxicity evaluation test (that is, an in vitro or in vivo toxicity evaluation test) using an actual chemical substance.

The client terminal 12 is a terminal used by the user 18. The server 14 receives a processing request from the client terminal 12 via the network 16 and provides a service corresponding to the request to the client terminal 12 that has made the request via the network 16. For example, in a case where the server 14 receives a request to execute processing related to a characteristic evaluation of a chemical substance as the processing request, the server 14 transmits an evaluation result to the client terminal 12. In the present embodiment, the server 14 is an example of an "information processing apparatus" according to the technology of the present disclosure.

For example, the server 14 is realized by a mainframe, but this is merely an example. For example, the server may be realized by cloud computing, or may be realized by network computing such as fog computing, edge computing, or grid computing. Here, the server 14 is exemplified as an example of a device provided outside the client terminal 12, but this is merely an example, and at least one personal computer or the like may be used instead of the server 14.

The network 16 is configured by, for example, at least one of a wide area network (WAN) or a local area network (LAN). Further, a connection method between the client terminal 12 and the network 16 and a connection method between the server 14 and the network 16 may be a wireless communication method or a wired communication method. The network 16 establishes communication between the client terminal 12 and the server 14, and transmits and receives various types of information between the client terminal 12 and the server 14.

A reception device 20 is connected to the client terminal 12. The reception device 20 receives an instruction from the user 18. The reception device 20 includes a keyboard 21, a mouse 22, and the like. The keyboard 21 and the mouse 22 shown in FIG. 1 are merely an example. As the reception device 20, any one of the keyboard 21 or the mouse 22 may be provided. In addition, as the reception device 20, for example, at least one of a proximity input device that receives a proximity input, a voice input device that receives a voice input, or a gesture input device that receives a gesture input may be applied instead of the keyboard 21 and/or the mouse 22. The proximity input device is, for example, a touch panel or a tablet.

A display device 24 is connected to the client terminal 12. Examples of the display device 24 include an electro-luminescence (EL) display and a liquid crystal display. The display device 24 displays various types of information (for example, image and text) under the control of the client terminal 12.

Here, a personal computer is described as an example of the client terminal 12, but this is merely an example. As the client terminal 12, a mobile terminal, such as a smartphone and a tablet terminal, may be used. Here, although an example in which one client terminal 12 is connected to one server 14 via the network 16 has been described, this is merely an example. It is needless to say that the information processing system 10 may include a plurality of client terminals 12 and a plurality of servers 14.

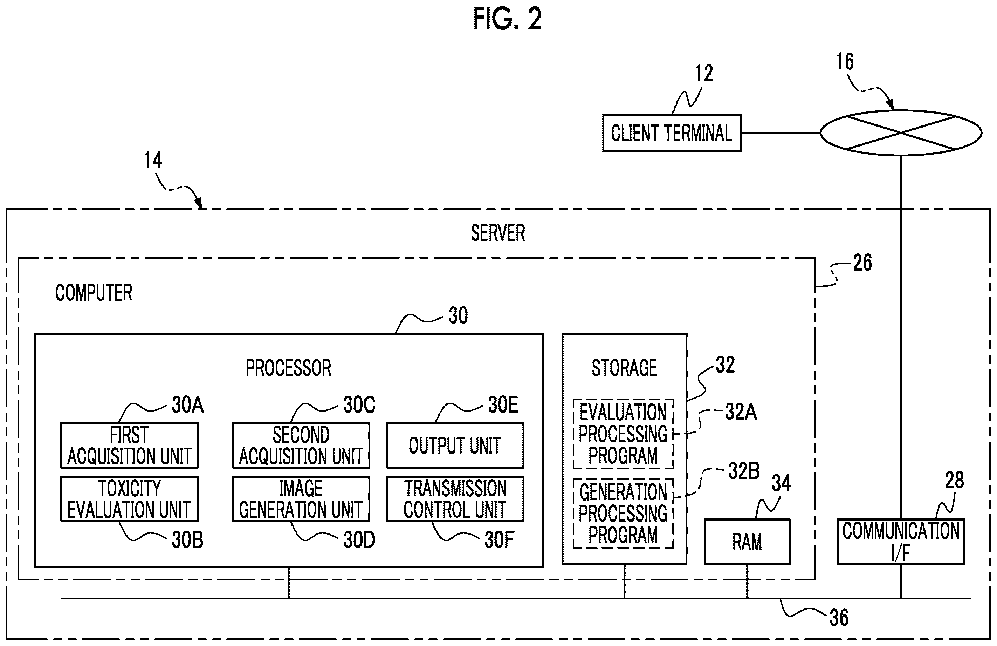

As shown in FIG. 2 as an example, the server 14 comprises a computer 26, a communication interface (I/F) 28, and a bus 36. The computer 26 comprises a processor 30, a storage 32, and a random access memory (RAM) 34. The processor 30, the storage 32, the RAM 34, and the communication I/F 28 are connected to the bus 36. In the present embodiment, the computer 26 is an example of a "computer" according to the technology of the present disclosure, and the processor 30 is an example of a "processor" according to the technology of the present disclosure.

A memory is connected to the processor 30. The memory includes the storage 32 and the RAM 34. The processor 30 includes, for example, a central processing unit (CPU) and a graphics processing unit (GPU). The GPU operates under the control of the CPU, and is responsible for executing processing related to an image.

The storage 32 is a non-volatile storage device that stores various programs, various parameters, and the like. Examples of the storage 32 include a flash memory (for example, an electrically erasable and programmable read only memory (EEPROM) and a solid state drive (SSD)), and/or a hard disk drive (HDD). The flash memory and the HDD are merely examples, and at least one of the flash memory, the HDD, a magnetoresistive memory, or a ferroelectric memory may be used as the storage 32.

The RAM 34 is a memory that transitorily stores information, and is used as a work memory by the processor 30. Examples of the RAM 34 include a dynamic random access memory (DRAM) or a static random access memory (SRAM).

The communication I/F 28 is connected to the network 16. The communication I/F 28 is responsible for transmitting and receiving information to and from an external communication device (for example, the client terminal 12) via the network 16. For example, the communication I/F 28 transmits information in response to a request from the processor 30 to the external communication device via the network 16. In addition, the communication I/F 28 receives the information transmitted from the external communication device, and outputs the received information to the processor 30 via the bus 36.

An evaluation processing program 32A is stored in the storage 32. The evaluation processing program 32A is a program that provides an evaluation simulation of toxicity of a chemical substance. The processor 30 reads out the evaluation processing program 32A from the storage 32 and executes the read-out evaluation processing program 32A on the RAM 34 to perform toxicity evaluation processing. The toxicity evaluation processing is realized by the processor 30 operating as a first acquisition unit 30A and a toxicity evaluation unit 30B.

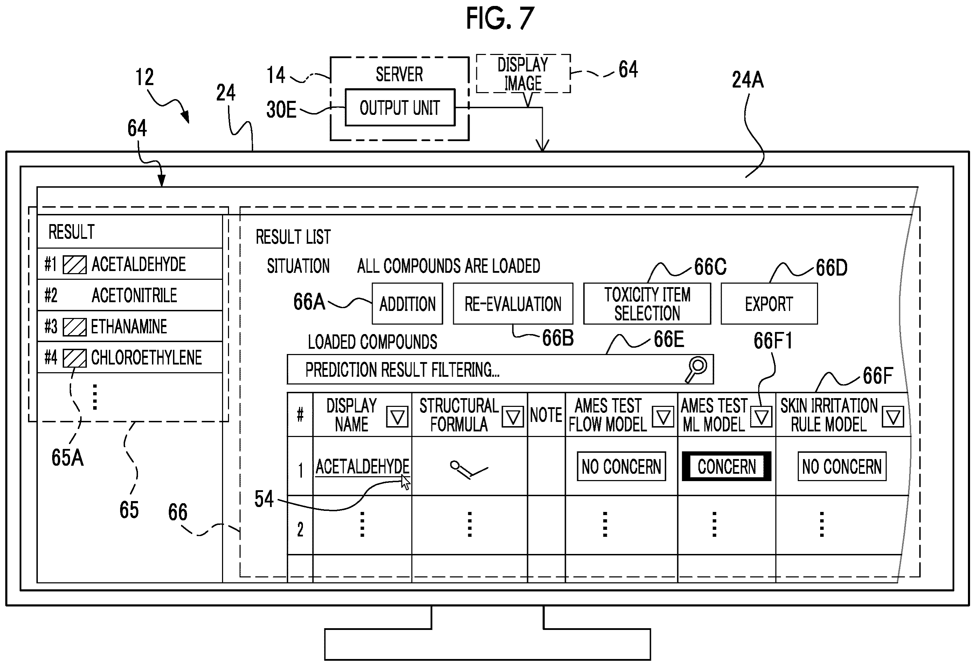

In addition, a generation processing program 32B is stored in the storage 32. The generation processing program 32B is a program that generates a display image 64 (see FIG. 7 and the like) to be output to the client terminal 12. The processor 30 reads out the generation processing program 32B from the storage 32 and executes the read-out generation processing program 32B on the RAM 34 to perform image generation processing. The image generation processing is realized by the processor 30 operating as a second acquisition unit 30C, an image generation unit 30D, an output unit 30E, and a transmission control unit 30F. In the present embodiment, the generation processing program 32B is an example of a "program" according to the technology of the present disclosure.

Here, a case where the user 18 uses the information processing system 10 to make an evaluation of toxicity to a human body (hereinafter, also simply referred to as a "toxicity evaluation") for a newly developed chemical substance A will be considered. Here, the toxicity refers to toxicity that the user 18 is interested in as an evaluation item, and examples thereof include mutagenicity, sensitization, and irritation. The mutagenicity refers to a property of causing an irreversible change in genetic information (a base sequence of deoxyribonucleic acid (DNA) or a structure or number of chromosomes) of an organism. The sensitization refers to a property of causing an allergic reaction upon exposure to a chemical substance. The irritation refers to a property in which a chemical substance or the like gives a stimulus (for example, pain or a burning sensation) to the tactile sense or the like.

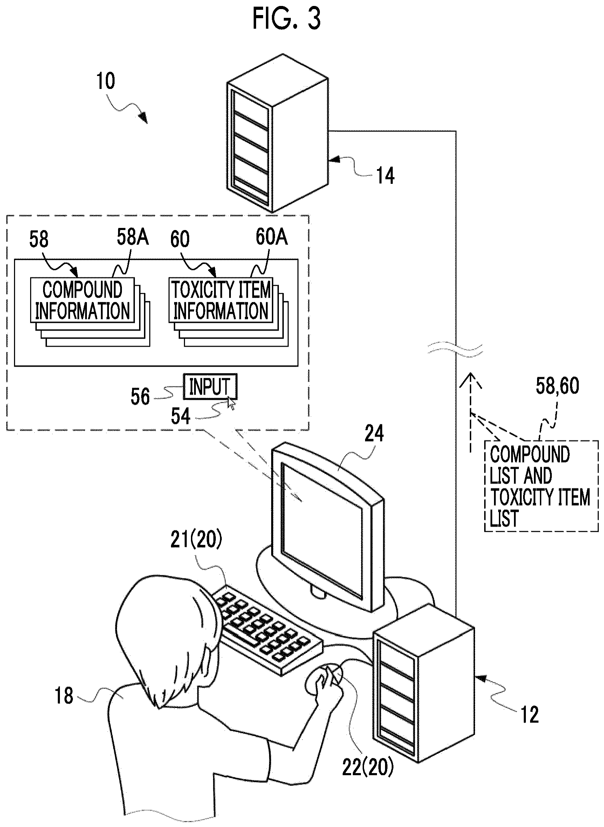

In a case of evaluating the toxicity, as shown in FIG. 3 as an example, the user 18 inputs a compound list 58 to the client terminal 12 via the reception device 20. The compound list 58 is a list of chemical substances that are targets of the toxicity evaluation. Examples of the chemical substance include a low-molecular-weight compound (for example, a compound having a molecular weight of less than 1000), a sugar, a peptide, a protein, and a nucleic acid. The compound list 58 includes a plurality of pieces of compound information 58A. The compound information 58A is information for specifying a chemical structure of the compound. More specifically, the compound information 58A is text information describing a chemical structure such as a structural formula, a compositional formula, and an amino acid sequence, or numerical information obtained by converting such text information into a numerical value. In addition, the compound list 58 may be a list of information indicating a chemical abstract service (CAS) number or a name of a chemical substance. In this case, the client terminal 12 or the server 14 refers to table data or the like in which a correspondence relationship between a CAS number or a name of a chemical substance and a chemical structure is recorded, and reads out the compound information 58A corresponding to the input chemical substance.

In addition, the compound list 58 may be a list of image data showing a chemical structure of a compound as a compound graph (that is, a data structure in which atoms are nodes and bonds are edges). In this case, the client terminal 12 or the server 14 refers to table data or the like in which a correspondence relationship between the compound graph and the compound information 58A is recorded, and reads out the compound information 58A corresponding to the selected compound graph.

In addition, the user 18 inputs a toxicity item list 60, which is a list of toxicity evaluation items, to the client terminal 12 via the reception device 20. Examples of the toxicity evaluation item include an evaluation item based on an AMES test and an evaluation item of skin irritation. The toxicity item list 60 includes a plurality of pieces of toxicity item information 60A. The toxicity item information 60A is information for specifying the toxicity evaluation item.

In the example shown in FIG. 3, a screen for selecting the compound list 58 and the toxicity item list 60 is displayed on a screen of the display device 24, and the user 18 performs a selection operation on the selection screen to select, for example, the compound list 58 and the toxicity item list 60. On the selection screen, for example, the user 18 clicks an input soft key 56 by operating a pointer 54 displayed on the screen of the display device 24 via the mouse 22. As a result, the compound list 58 and the toxicity item list 60 are transmitted from the client terminal 12 to the server 14, and a processing request is made regarding the toxicity evaluation for the compound.

Here, although an example of a form in which the plurality of pieces of compound information 58A are listed and input at once has been described, it is needless to say that each of the plurality of pieces of compound information 58A may be individually input or may be input by being divided into a plurality of lists. In addition, one piece of compound information 58A may be input, and the toxicity of one compound and metabolites may be evaluated.

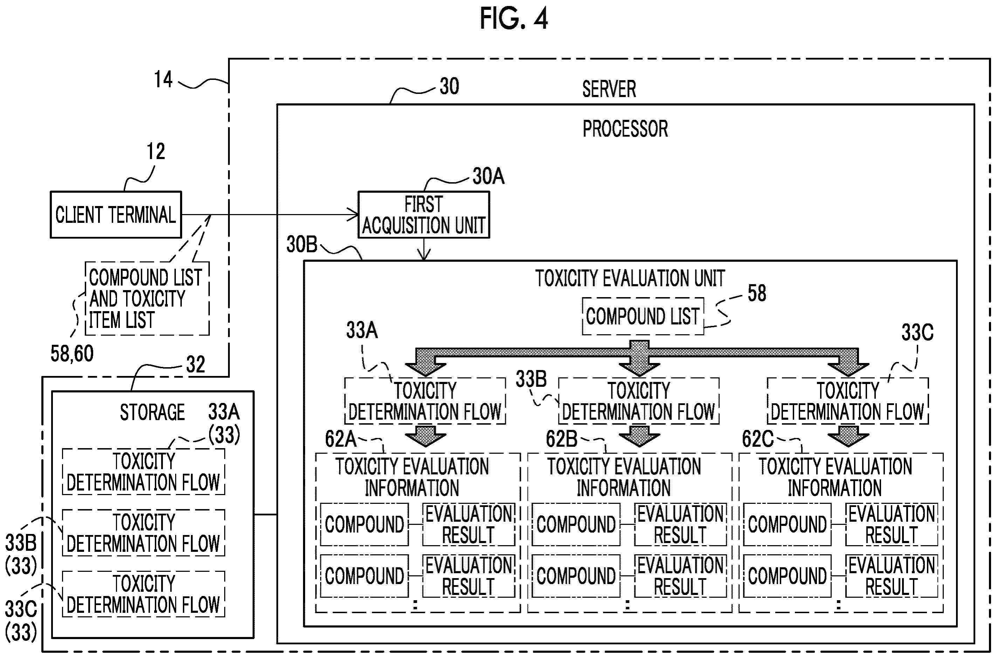

As shown in FIG. 4 as an example, the compound list 58 and the toxicity item list 60 are output from the client terminal 12 to the server 14. In the processor 30 of the server 14, the toxicity evaluation processing is executed. The toxicity evaluation processing is processing of evaluating the toxicity of a compound. In the toxicity evaluation processing, the first acquisition unit 30A outputs the acquired compound list 58 and toxicity item list 60 to the toxicity evaluation unit 30B. The toxicity evaluation unit 30B evaluates the toxicity of a plurality of compounds (that is, a compound group) indicated by the compound information 58A of the compound list 58.

Specifically, the toxicity evaluation unit 30B executes the toxicity evaluation processing according to a toxicity determination flow 33. The toxicity determination flow 33 is described in the evaluation processing program 32A, and is schematically shown in a form of being stored in the storage 32 in FIG. 4. The toxicity determination flow 33 is a determination flow used for evaluating the toxicity. The toxicity determination flow 33 has at least one determination step, and has a route that branches according to a determination result in the determination step. Determination items in each determination step are predetermined. Examples of the determination item include whether or not a compound disappears through metabolism, whether or not a compound permeates a cell membrane, and whether or not a compound corresponds to a structural-alert rule. The structural-alert rule is a regulation related to a chemical structure, and is a rule for specifying a partial structure that may have toxicity of interest to the user. The toxicity determination flow 33 is an example of a "determination flow" according to the technology of the present disclosure.

In addition, the toxicity determination flow 33 is linked to the toxicity evaluation item, and the number of determination steps and the determination content for each determination step are different for each toxicity evaluation item. In the example shown in FIG. 4, toxicity determination flows 33A to 33C corresponding to different evaluation items are displayed in a distinguishable manner as the toxicity determination flow 33.

The toxicity evaluation unit 30B acquires the toxicity item information 60A included in the toxicity item list 60. Then, the toxicity evaluation unit 30B selects the toxicity determination flows 33A to 33C according to the toxicity evaluation items indicated by the toxicity item information 60A.

The toxicity evaluation unit 30B inputs the compound list 58 to each of the toxicity determination flows 33A to 33C. The toxicity evaluation unit 30B executes a determination regarding toxicity evaluation for each determination step on the compound information 58A in each of the toxicity determination flows 33A to 33C. The toxicity evaluation unit 30B outputs toxicity evaluation information 62A to 62C as toxicity evaluation results according to the respective toxicity determination flows 33A to 33C to be executed. Steps ST1 to ST5 described below are examples of a "determination step" according to the technology of the present disclosure.

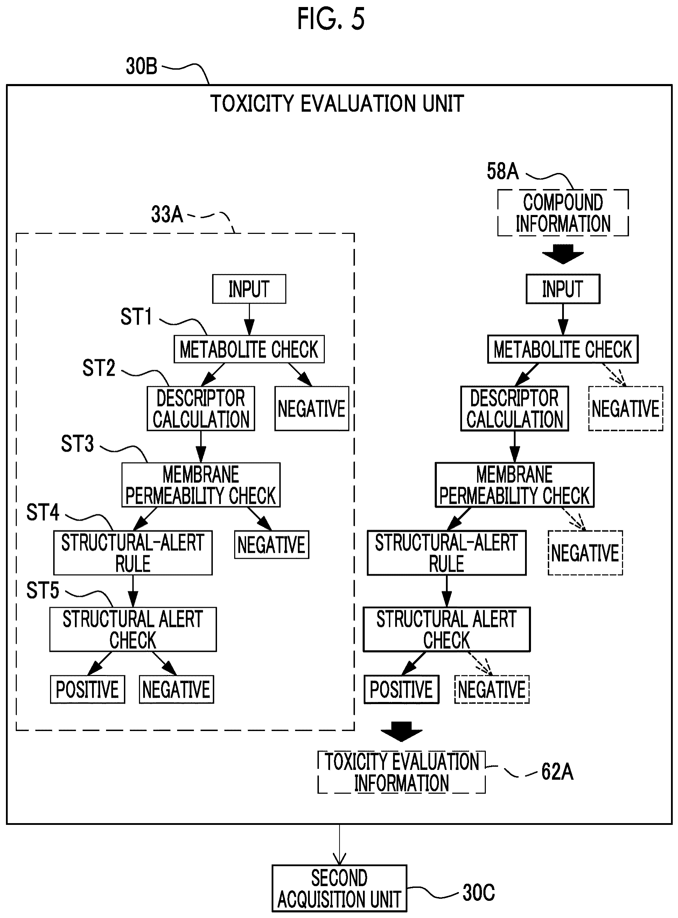

In the following description of the toxicity determination flow 33, the toxicity determination flow 33A will be described as an example. As shown in FIG. 5 as an example, the compound information 58A is input to the toxicity determination flow 33A. In the toxicity determination flow 33A, in step ST1, it is determined whether or not the compound indicated by the compound information 58A is a compound that persists in the body without being metabolically degraded. In a case where the determination in step ST1 is affirmative, the toxicity evaluation for the compound proceeds to step ST2. In a case where the determination in step ST1 is negative, as a result of the toxicity evaluation for the compound, it is determined that the compound is a compound (hereinafter, also simply referred to as a "disappearance compound") that is degraded and disappears in the body, and a negative (that is, non-toxic) evaluation result is output.

Here, in a metabolic reaction, a metabolic enzyme forms a complex with a compound that fits into an active site of the metabolic enzyme. The metabolic enzyme has an action of promoting a metabolic reaction by lowering the energy (that is, the activation energy) required for the compound to cause a chemical reaction. The term "disappearance compound" refers to a compound that, in a case of forming a complex with a metabolic enzyme, has lower activation energy than other metabolites, undergoes a chemical reaction more rapidly than other metabolites, and is converted into the next metabolite in a short period of time. Therefore, the disappearance compound is less likely to react with DNA in a cell, and the necessity of evaluating toxicity (that is, positive or negative evaluation) is low.

In step ST2, descriptor calculation is executed on the compound information 58A. Examples of the descriptor include a molar mass or a LogP (a value obtained by taking the common logarithm of an octanol/water partition coefficient) of the compound. After the process of step ST2 is executed, the toxicity evaluation proceeds to step ST3.

In step ST3, it is determined whether or not the compound can permeate the cell membrane based on a result of the descriptor calculation of the compound executed in step ST2. The numerical value indicated by the descriptor is compared with a threshold value, and a determination is made as to whether or not the compound can permeate the cell membrane based on a comparison result. In a case where the determination in step ST3 is affirmative, the toxicity evaluation proceeds to step ST4. In a case where the determination in step ST3 is negative, a negative (that is, non-toxic) evaluation result is output as the result of the toxicity evaluation. This is because the compound does not permeate the cell membrane and is therefore considered to have little effect on the body.

In step ST4, the structural-alert rule is applied to the compound information 58A. After the process of step ST4 is executed, the toxicity evaluation proceeds to step ST5.

In step ST5, it is determined whether or not a molecular structure indicated by the compound information 58A includes a structural alert. In a case where the determination in step ST5 is negative, a negative (that is, non-toxic) evaluation result is output as the result of the toxicity evaluation. This is because the compound does not have a structural alert and is therefore considered to have a low likelihood of exhibiting toxicity.

In a case where the determination in step ST5 is affirmative, a positive (that is, toxic) evaluation result is output as the result of the toxicity evaluation. In the example shown in FIG. 5, paths and determination steps taken in the toxicity determination flow 33A are shown as the toxicity evaluation for the compound, and an example in which the compound is finally evaluated as positive is shown. In addition, the toxicity evaluation information 62A includes information indicating the result of the toxicity evaluation as well as information indicating a path (hereinafter, also simply referred to as an "evaluation path") leading to the result of the toxicity evaluation in the toxicity determination flow 33A, information indicating an explanation of the determination item, and information indicating details of a determination result for each determination step.

As described above, the toxicity determination flow 33A outputs the toxicity evaluation information 62A according to the input compound information 58A. In the toxicity evaluation information 62, the evaluation result is linked to each compound (see FIG. 4). In this way, the toxicity evaluation processing is executed. The toxicity evaluation unit 30B outputs the toxicity evaluation information 62 to the second acquisition unit 30C.

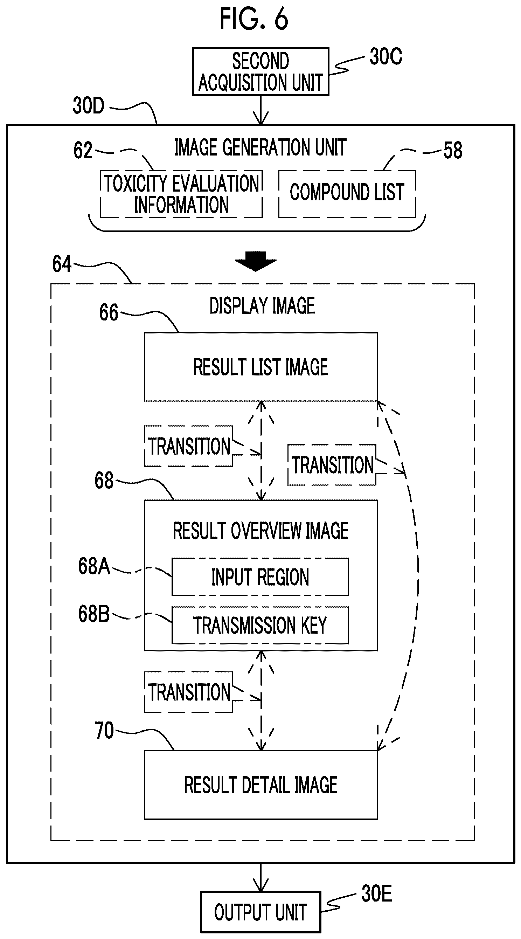

As shown in FIG. 6 as an example, the second acquisition unit 30C outputs the acquired toxicity evaluation information 62 and compound list 58 to the image generation unit 30D. The image generation unit 30D generates a display image 64 based on the toxicity evaluation information 62 and the compound list 58. The display image 64 is an image in which the result of the toxicity evaluation can be displayed. The display image 64 includes a result list image 66, a result overview image 68, and a result detail image 70. The result list image 66 is an image showing a list of compounds together with an evaluation result of each compound. The result overview image 68 is an image showing an overview of evaluation results of toxicity items of a compound. In addition, the result detail image 70 is an image showing details of evaluation results of the toxicity items of a compound.

The result list image 66, the result overview image 68, and the result detail image 70 can transition from one another. That is, in a case where a certain compound is selected in the result list image 66, a transition is made to the result overview image 68 for the compound. Then, in a case where a certain toxicity item is selected in the result overview image 68, a transition is made to the result detail image 70 for the toxicity item. In addition, a transition between the result detail image 70 and the result list image 66 is also possible. The result list image 66 is an example of a "list image" according to the technology of the present disclosure.

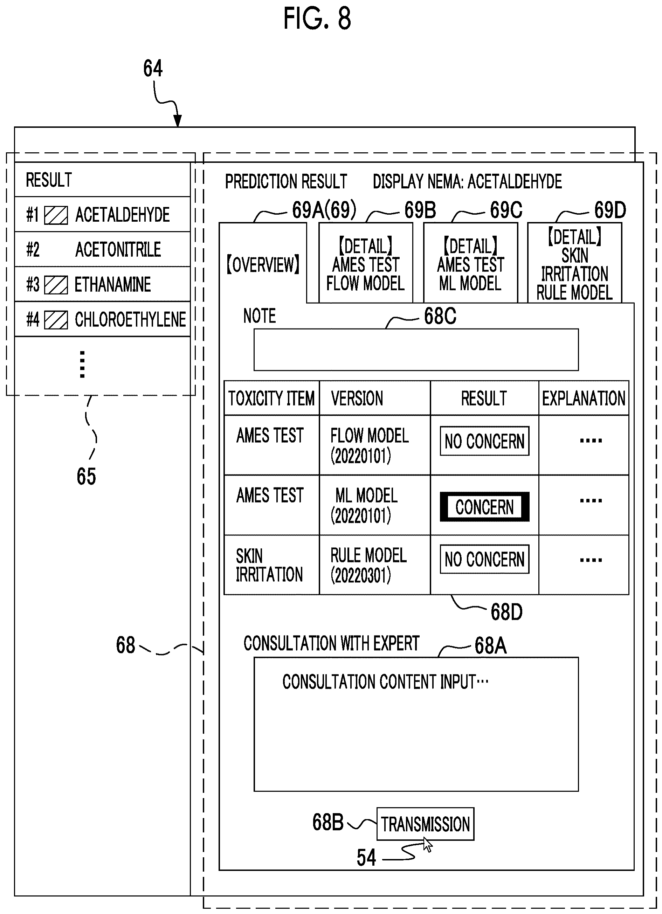

In addition, the result overview image 68 includes an input region 68A and a transmission key 68B (see also FIG. 8). The input region 68A is a region in which the inquiry content regarding the evaluation result of the toxicity item can be input. In addition, the transmission key 68B is a soft key for receiving an operation of transmitting an inquiry notification regarding the evaluation result of the toxicity item to a designated destination. The result overview image 68 is an example of an "inquiry image" according to the technology of the present disclosure, and the transmission key 68B is an example of an "operation unit" according to the technology of the present disclosure. In addition, the input region 68A is an example of an "input region" according to the technology of the present disclosure.

The image generation unit 30D outputs the generated display image 64 to the output unit 30E. As shown in FIG. 7 as an example, the output unit 30E of the server 14 executes control of outputting the display image 64 to the client terminal 12. In other words, the server 14 transmits information indicating the display image 64 to the client terminal 12. On the display device 24 of the client terminal 12, a screen 24A including the display image 64 is displayed. The display device 24 is an example of a "display unit" according to the technology of the present disclosure.

A sidebar image 65 is displayed at a left end portion of the display image 64. The sidebar image 65 shows a list of compounds to be evaluated. In the example shown in FIG. 7, in the sidebar image 65, "acetaldehyde, acetonitrile, ethanamine, and chloroethylene" are shown as the compounds to be evaluated. In addition, a mark 65A indicating that the result of the toxicity evaluation of the compound is "positive" is displayed on a left side of the compound name. In the display image 64, even in a case where the result list image 66, the result overview image 68, and the result detail image 70 transition from one another, the sidebar image 65 is always displayed. Therefore, it is easy for the user 18 to check the list of evaluation results.

The result list image 66 is displayed on a right side of the sidebar image 65 of the display image 64. The result list image 66 comprises an addition key 66A, a re-evaluation key 66B, an item selection key 66C, and an export key 66D. The addition key 66A is a soft key that is operated in a case of adding the compound information 58A indicating the compound to be evaluated. The re-evaluation key 66B is a soft key for re-evaluating the toxicity in a case where the compound information 58A is changed or the toxicity item is changed. The item selection key 66C is a soft key for changing the toxicity item. The export key 66D is a soft key for outputting the evaluation result to the outside.

In addition, a search bar 66E is displayed in the result list image 66. By inputting a text into the search bar 66E, compounds that match the text are filtered out from the compounds to be evaluated. The text that can be input for the search may be, for example, a name of a compound or a character string describing a CAS number or a molecular structure.

In the result list image 66, a result list table 66F showing a list of the evaluation results of the toxicity items for each compound is shown. In the example shown in FIG. 7, items in the columns of the result list table 66F are "#", "display name", "structural formula", "note", "AMES test flow model", "AMES test ML model", and "skin irritation rule model" in order from the left. In each row of the result list table 66F, contents corresponding to the items of the columns are described. In the example shown in FIG. 7, the content regarding acetaldehyde of #1 (for example, the structural formula and the evaluation result of the toxicity) is described.

In a first row of the result list table 66F, a filter button 66F1 is provided in each column. Each column can be sorted in ascending or descending order by operating the filter button 66F1. As described above, in the result list table 66F, the compounds displayed in the result list table 66F can be filtered out and/or sorted according to a condition designated by the user 18 by using the search bar 66E and/or the filter button 66F1.

In addition, by selecting a display name in the sidebar image 65 and the result list table 66F, the result list image 66 transitions to the result overview image 68. In the example shown in FIG. 7, the display name "acetaldehyde" in the result list table 66F is selected via the pointer 54, and a transition to the result overview image 68 related to acetaldehyde is shown.

As shown in FIG. 8 as an example, in the display image 64, the result list image 66 transitions to the result overview image 68. In the example shown in FIG. 8, the result overview image 68 related to acetaldehyde is shown. The result overview image 68 includes a note input region 68C. The note input region 68C is a region in which the user 18 can input any text. The content input in the note input region 68C is reflected in a cell of the column "note" of the result list table 66F (see FIG. 7) of the result list image 66.

In addition, a result overview table 68D is shown in the result overview image 68. The result overview table 68D is a table showing the evaluation result for each toxicity item for the selected compound. In the example shown in FIG. 8, items in the columns of the result overview table 68D are "toxicity item", "version", "result", and "explanation" in order from the left. In each row of the result overview table 68D, contents corresponding to the items of the columns are described. In the example shown in FIG. 8, results of three types of toxicity items, namely, the AMES test (flow model and machine learning (ML) model) and skin irritation (rule model), are shown. Here, evaluation results for the toxicity of acetaldehyde show that there is no concern for the AMES test (flow model) and skin irritation (rule model), while there is concern for the AMES test (ML model).

The result overview image 68 includes the input region 68A and the transmission key 68B. The user 18 inputs the inquiry content regarding the evaluation result to the input region 68A. Here, the input region 68A is a text input region (for example, an email form) in which a text indicating the inquiry content can be input. Then, by operating the transmission key 68B, an inquiry notification including the inquiry content is transmitted to a destination (for example, a client terminal of an expert) designated in advance. The inquiry content may include a cause of the evaluation result displayed in the result overview image 68, or a cause of a difference in evaluation results for each toxicity item (for example, a difference in evaluation results of the AMES test (flow model and ML model)).

In addition, a tab 69 is displayed in an upper part of the result overview image 68. In a case where the tab 69 is selected, the result overview image 68 and the result detail image 70 related to each toxicity item can be switched. In the example shown in FIG. 8, tabs 69A to 69D are displayed. The tab 69A corresponds to the result overview image 68, the tab 69B corresponds to the result detail image of the AMES test flow model, the tab 69C corresponds to the result detail image of the AMES test ML model, and the tab 69D corresponds to the result detail image of the skin irritation rule model.

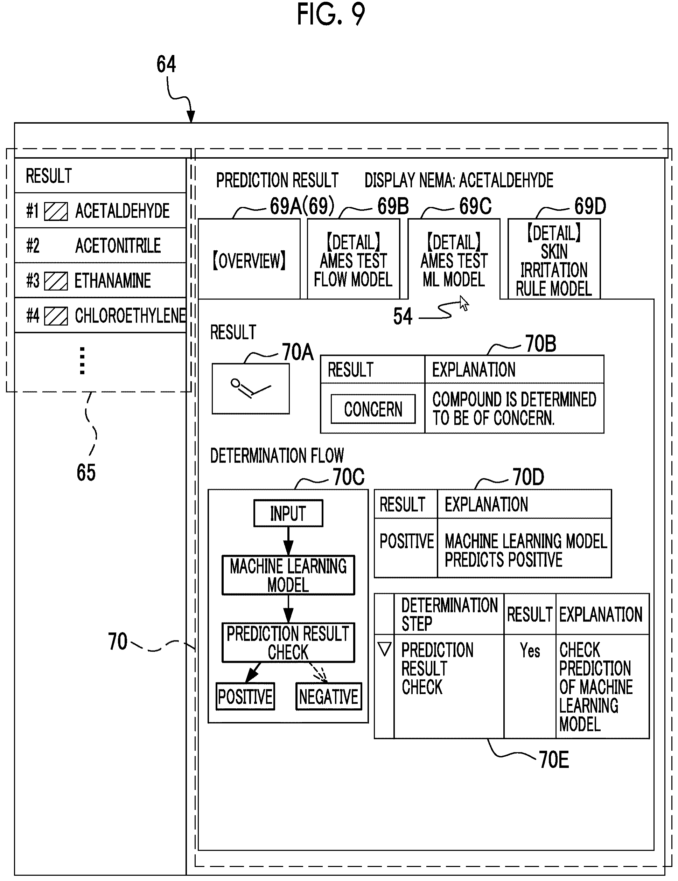

As shown in FIG. 9 as an example, the result overview image 68 transitions to the result detail image 70 in the display image 64 by selecting the tab 69 corresponding to the result detail image. In the example shown in FIG. 9, by selecting the tab 69C via the pointer 54, the result detail image 70 related to a result of the toxicity evaluation of acetaldehyde using the AMES test ML model is displayed.

The result detail image 70 includes a structural formula image 70A showing a structural formula of the compound and a final result image 70B showing a final result of the toxicity evaluation. In the example shown in FIG. 9, a structural formula of acetaldehyde is shown in the structural formula image 70A, and "concern" as a result and "compound is determined to be of concern" as an explanation are described in the final result image 70B.

In addition, the result detail image 70 includes a flow image 70C showing the toxicity determination flow 33 used for the toxicity evaluation of the compound, a result explanation image 70D for explaining the evaluation result in the toxicity determination flow 33, and a determination step explanation image 70E for explaining the determination content for each determination step included in the toxicity determination flow 33. The toxicity determination flow 33 is an example of a "determination flow" according to the technology of the present disclosure.

In the flow image 70C, a path (that is, an evaluation path) leading to the evaluation result is displayed. In the example shown in FIG. 9, an evaluation path (here, a path leading to "positive") of acetaldehyde in the AMES test ML model is displayed. In addition, in the flow image 70C, the evaluation path is shown to be distinguishable from other paths in the toxicity determination flow 33. In the example shown in FIG. 9, the evaluation path is indicated by a thick line, and the other paths are indicated by a broken line.

In the example shown in FIG. 9, in the determination step explanation image 70E, "prediction result check" is shown as the determination step, and an explanation of the determination content of "check prediction of machine learning model" is shown as the corresponding explanation. In addition, in the result explanation image 70D, "positive" is shown as the result of the toxicity evaluation, and an explanation of the evaluation result that "machine learning model predicts positive" is shown.

In this way, the user 18 can understand a list of toxicity evaluation results of a plurality of compounds and an overview and details of the toxicity evaluation of the selected compound by visually recognizing the result list image 66, the result overview image 68, and the result detail image 70. That is, by checking the evaluation results while transitioning between the result list image 66, the result overview image 68, and the result detail image 70 in the display image 64, the toxicity evaluation of the compound group can be checked from an overall perspective to a detailed perspective.

Incidentally, in the toxicity evaluation of the compound, it may be necessary to further examine the results, in addition to simply checking the evaluation results. In this case, in a case where it is difficult for the user 18 alone to interpret the evaluation result (for example, in a case where it is unclear why such an evaluation result is obtained, or in a case where the validity or reliability of the evaluation result is unclear), the user 18 needs to share compound structural information and the like with an expert (for example, a person or a department in charge of safety management of chemical substances within the company) or an external specialized organization (hereinafter, simply referred to as an "expert") and explain the situation. In this case, for example, in a case where the evaluation result is explained only verbally or a document related to the evaluation result is separately created in order to share information with the expert, the workload on the user 18 is increased. In addition, explanations given only verbally can easily lead to miscommunication, such as the inquiry content not being conveyed accurately.

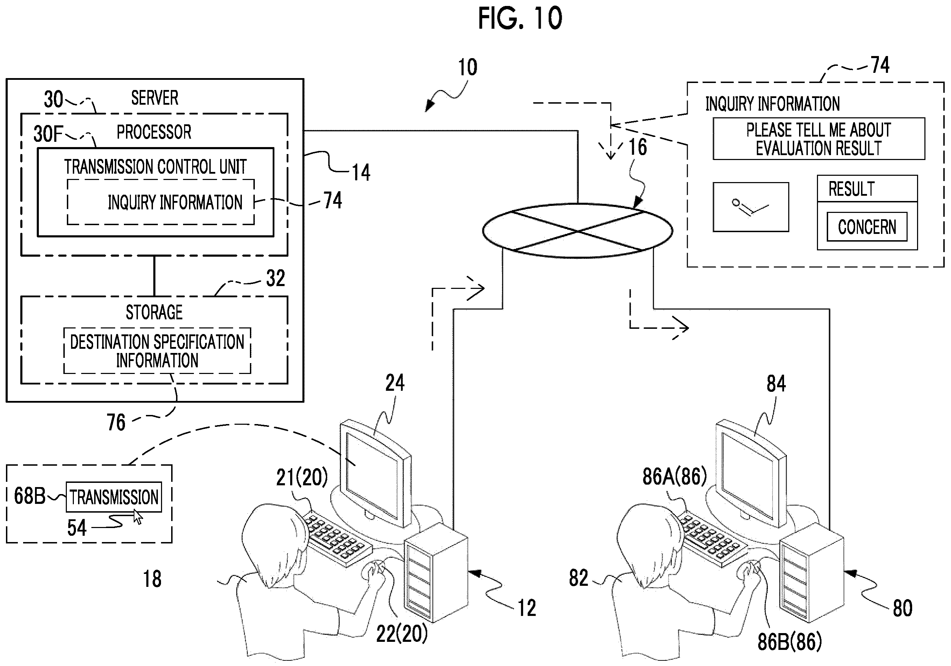

Therefore, in the information processing system 10 according to the present embodiment, control of transmitting an inquiry notification including information that the user 18 wishes to share with the expert regarding the toxicity evaluation result is executed. In this case, the user 18 inputs the inquiry content to the input region 68A displayed in the result overview image 68 (see FIG. 8). As shown in FIG. 10 as an example, in a case where the transmission key 68B is clicked via the pointer 54, inquiry information 74, which is information indicating the inquiry notification, is transmitted from the client terminal 12 to the server 14. Then, in the server 14, the transmission control unit 30F of the processor 30 executes control of transmitting the inquiry information 74 to a destination designated in advance. Specifically, destination specification information 76 is stored in the storage 32 of the server 14. The destination specification information 76 is information for specifying a destination that is designated in advance as a destination to which the evaluation result is to be shared. The destination specification information 76 is specifically a destination address of an expert 82. The destination address is, for example, an account name or an e-mail address for each expert 82 set on the server 14. In addition, the destination address may be an identification (ID) of a terminal used by the expert 82.

The transmission control unit 30F transmits the inquiry information 74 to the destination indicated by the destination specification information 76. In the example shown in FIG. 10, the inquiry notification is transmitted to the ID specified by the destination specification information 76 by using a message transmission/reception function in the information processing system 10. In the inquiry notification indicated by the inquiry information 74, there is a description of "please tell me about evaluation result" as the inquiry content, and a structural formula of the compound to be evaluated and a result of "concern" as the evaluation result are shown.

The inquiry information 74 transmitted from the server 14 is received by a client terminal 80 under the control of the transmission control unit 30F. The client terminal 80 is a terminal device used by the expert 82. The expert 82 visually recognizes the content of the inquiry notification indicated by the inquiry information 74 via a display device 84. This allows the user 18 and the expert 82 to share information regarding the evaluation result. Then, the expert 82 answers the inquiry of the user 18 by inputting an answer to the inquiry content via, for example, a reception device 86 (here, a keyboard 86A and a mouse 86B) and transmitting information indicating the answer.

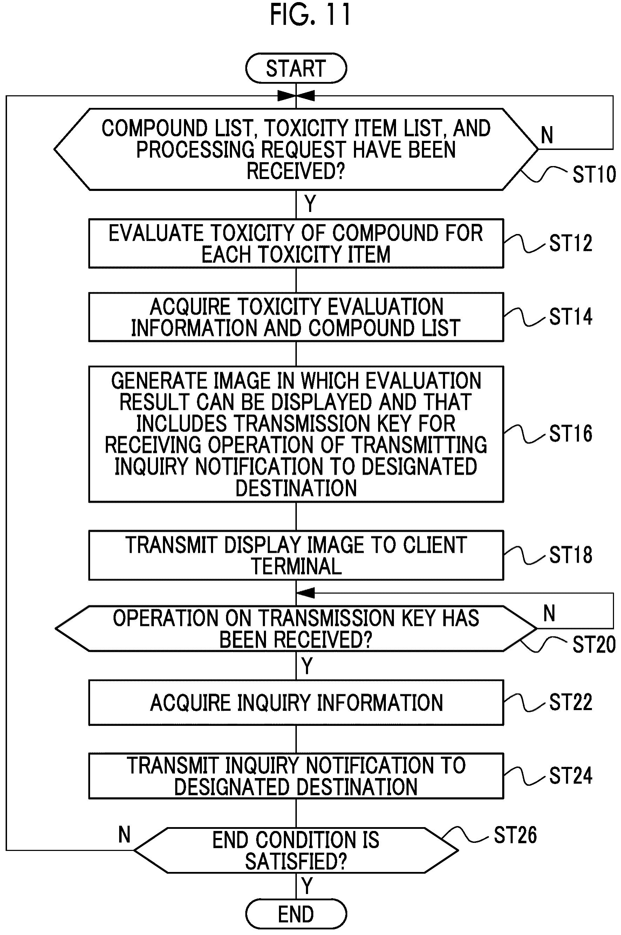

Next, control processing of the server 14 according to the present embodiment will be described with reference to FIG. 11. The server control processing is processing executed by the server 14, and includes the above-described toxicity evaluation processing and image generation processing. FIG. 11 is a flowchart showing an example of the server control processing. The flow of the processing shown in FIG. 11 is an example of an "information processing method" according to the technology of the present disclosure.

In the server control processing shown in FIG. 11 as an example, first, in step ST10, the first acquisition unit 30A determines whether or not the compound list 58, the toxicity item list 60, and the processing request have been acquired. In a case where it is determined in the determination in step ST10 that the compound list 58, the toxicity item list 60, and the processing request have been acquired, the determination is affirmative, and the server control processing proceeds to step ST12. In a case where it is determined in the determination in step ST10 that the compound list 58, the toxicity item list 60, and the processing request have not been acquired, the determination is negative, and the server control processing returns to step ST10.

In step ST12, the toxicity evaluation unit 30B performs the toxicity evaluation on each of the compounds indicated by the compound list 58 based on the toxicity item list 60 acquired in step ST10. Specifically, the toxicity evaluation unit 30B inputs the compound information 58A to the toxicity determination flow 33 according to the toxicity item indicated by the toxicity item information 60A. The toxicity determination flow 33 outputs the toxicity evaluation information 62 for each compound. The toxicity evaluation unit 30B acquires the toxicity evaluation information 62. After the process of step ST12 is executed, the server control processing proceeds to step ST14.

In step ST14, the second acquisition unit 30C acquires the toxicity evaluation information 62 and the compound list 58. After the process of step ST14 is executed, the server control processing proceeds to step ST16.

In step ST16, the image generation unit 30D generates a display image 64 based on the compound list 58 and the toxicity evaluation information 62 acquired in step ST14. The display image 64 is an image in which the result of the toxicity evaluation can be displayed. In addition, the display image 64 includes the result overview image 68, and the input region 68A and the transmission key 68B are displayed in the result overview image 68. After the process of step ST16 is executed, the server control processing proceeds to step ST18.

In step ST18, the output unit 30E executes control of outputting the display image 64 generated by the image generation unit 30D in step ST18 to the client terminal 12. Specifically, the output unit 30E transmits the display image 64 to the client terminal 12. After the process of step ST18 is executed, the server control processing proceeds to step ST20.

In step ST20, the transmission control unit 30F determines whether or not an operation on the transmission key 68B has been received. In a case where it is determined in the determination in step ST20 that the operation on the transmission key 68B has been acquired, the determination is affirmative, and the server control processing proceeds to step ST22. In a case where it is determined in the determination in step ST20 that the operation on the transmission key 68B has not been acquired, the determination is negative, and the server control processing returns to step ST20.

In step ST22, the transmission control unit 30F acquires the inquiry information 74 from the client terminal 12. After the process of step ST22 is executed, the server control processing proceeds to step ST24.

In step ST24, the transmission control unit 30F executes control of transmitting the inquiry information 74 to the destination indicated by the destination specification information 76. After the process of step ST24 is executed, the server control processing proceeds to step ST26.

In step ST26, the transmission control unit 30F determines whether or not a condition (hereinafter, referred to as an "end condition") for ending the server control processing is satisfied. Examples of the end condition include a condition in which an instruction to end the server control processing is received. In step ST26, in a case where the end condition is not satisfied, the determination is negative, and the server control processing proceeds to step ST10. In step ST26, in a case where the end condition is satisfied, the determination is affirmative, and the server control processing ends.

As described above, in the information processing system 10 according to the present embodiment, the toxicity evaluation of the compound is performed by the toxicity evaluation unit 30B in the processor 30 of the server 14. Then, the image generation unit 30D generates a display image 64. The display image 64 is an image in which the toxicity evaluation result of the compound can be displayed. In addition, the display image 64 includes the result overview image 68, and the result overview image 68 includes the transmission key 68B for receiving an operation of transmitting an inquiry notification regarding the evaluation result to the expert 82. The output unit 30D outputs the display image 64 to the display device 24 of the client terminal 12. In a case where the transmission key 68B is operated, the transmission control unit 30F executes control of transmitting the inquiry information 74 that can be shared with the expert 82 and that includes the evaluation result of the toxicity of the compound. As a result, by clicking the transmission key 68B included in the display image 64 showing the toxicity evaluation of the compound, the information regarding the toxicity evaluation is transmitted, so that it is easy to share the information between the user 18 and the expert 82. As a result, this configuration makes it possible to support the user in evaluating the toxicity of the compound.

For example, the known toxicity evaluation software in the related art does not have a function for sharing information, so users have had to use another means to contact an expert (for example, manually transcribing the toxicity evaluation result or verbally explaining the toxicity evaluation result). In this case, the information contained in the toxicity evaluation software could not be shared directly with the expert, so that no consultation was possible. Therefore, there was room for improvement in expert review of unknown compounds in particular. In this configuration, since the toxicity evaluation result obtained by the information processing system 10 can be shared directly with a predetermined destination, the effort required for the user 18 to perform operations is reduced, and information sharing becomes easier.

In addition, in the information processing system 10 according to the present embodiment, the display image 64 includes the result list image 66 showing a result of performing the toxicity evaluation on each of compounds included in a plurality of compound groups as toxicity evaluation targets. Then, the result overview image 68 is displayed by transitioning from the result list image 66 in a case where the compound is selected in the result list image 66. As a result, even in a case where there are a large number of compounds that are targets of the toxicity evaluation, it is possible to determine whether or not to share information with the expert 82 after checking the evaluation content of each of the plurality of compounds.

In addition, in the information processing system 10 according to the present embodiment, the result list image 66 includes the search bar 66E. As a result, in the result list image 66, the compounds to be evaluated are filtered out. In addition, the filter button 66F1 is provided in the result list table 66F included in the result list image 66. As a result, the description contents of each column of the result list table 66F are sorted in descending order or ascending order. As a result, even in a case where there are a large number of compounds that are targets of the toxicity evaluation, it is possible to determine whether or not to share information with the expert 82 after checking the content of the evaluation result for the compound of interest of the user 18.

In addition, in the information processing system 10 according to the present embodiment, the result overview image 68 includes the input region 68A in which the inquiry content for the evaluation result can be input. The content input in the input region 68A is included in the inquiry information 74 to be transmitted to the expert 82. This allows the inquiry information 74 to include the inquiry content input by the user 18, making it possible for the user 18 to inquire of the expert 82 about the content that he or she wishes to know.

In addition, in the information processing system 10 according to the present embodiment, the input region 68A included in the result overview image 68 is a text input region in which a text indicating the inquiry content can be input. Accordingly, a text freely input by the user 18 can be included in the inquiry information 74. As a result, as compared to a case where the type of the inquiry content is predetermined, the user 18 can accurately inquire about the content that he or she wishes to know.

The flow of the toxicity evaluation in the toxicity determination flow 33 shown in the first embodiment is merely an example. The flow of the toxicity evaluation in the toxicity determination flow 33 can be appropriately selected or changed by the user. In addition, the determination steps shown in the flow image 70C of the result detail image 70 do not necessarily have to be all of the determination steps included in the toxicity determination flow 33, and may be in a form in which some of the determination steps are displayed. For example, the determination step of interest of the user 18 may be displayed as the flow image 70C.

In addition, in the first embodiment, although an example of a form in which the result overview image 68 and the result detail image 70 related to each toxicity item can be switched by selecting the tab 69 has been described, the technology of the present disclosure is not limited to this. For example, the result overview image 68 may transition to the result detail image 70 related to each toxicity item by clicking the toxicity item of the result overview table 68D shown in the result overview image 68.

In addition, in the first embodiment, although an example of a form in which the transition to the result overview image 68 related to the compound corresponding to the display name is made by selecting the display name of the result list table 66F has been described, the technology of the present disclosure is not limited to this. For example, by selecting the evaluation result of each toxicity item in the result list table 66F, the result list image 66 may transition directly to the result detail image 70 related to each toxicity item without passing through the result overview image 68.

In addition, in the first embodiment, although an example of a form in which the inquiry content is input by inputting the text to the input region 68A has been described, the technology of the present disclosure is not limited to this. For example, as a method of inputting the inquiry content, the inquiry content may be input by selecting a radio button or a check button corresponding to predetermined content.

In addition, in the first embodiment, although an example of a form in which the inquiry notification is transmitted by using the message transmission/reception function in the information processing system 10, that is, the dedicated message transmission/reception function incorporated in the information processing system 10 has been described, the technology of the present disclosure is not limited to this. For example, the inquiry notification may be transmitted by using external message transmission/reception software separate from the information processing system 10, such as commercially available email software. In this case, the control of transmitting the inquiry notification executed by the transmission control unit 30F is, for example, control of activating external email software or chat software installed on the client terminal 12, transcribing the inquiry content into an email or chat, and then transmitting the email or the like.

### (First Modification Example)

In the first embodiment, although an example of a form has been described in which, in the inquiry notification, there is a description of "please tell me about evaluation result" as the inquiry content, and a structural formula of the compound to be evaluated and a result of "concern" as the evaluation result are shown, the technology of the present disclosure is not limited to this. In the present first modification example, the inquiry notification includes the toxicity evaluation result for each toxicity item, the determination flow, and the explanation of the determination step.

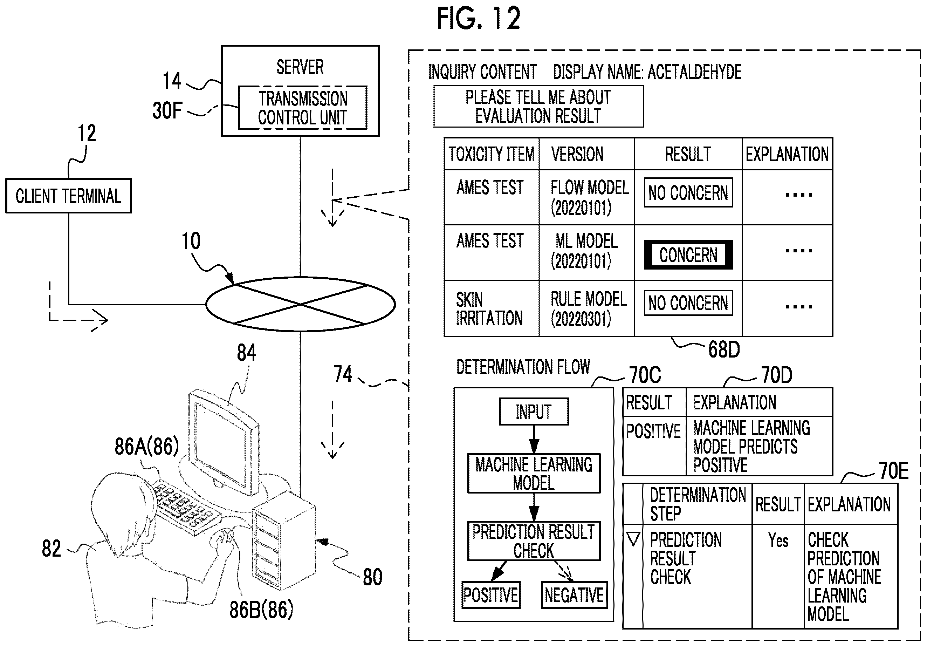

As shown in FIG. 12 as an example, the inquiry information 74, which is information indicating the inquiry notification, is transmitted from the client terminal 12 to the server 14. Then, in the server 14, the transmission control unit 30F of the processor 30 executes control of transmitting the inquiry information 74 to the client terminal 80. In the example shown in FIG. 12, an example is shown in which the inquiry information 74 indicating the inquiry notification regarding the evaluation result of acetaldehyde is transmitted.

The result overview table 68D is shown in the inquiry notification indicated by the inquiry information 74. The result overview table 68D shows the evaluation result for each toxicity item. In addition, the inquiry notification includes the flow image 70C. In the flow image 70C, the evaluation path is shown to be distinguishable from other paths in the toxicity determination flow 33. Here, an evaluation path of acetaldehyde in the AMES test ML model is shown.

In addition, the inquiry notification includes the determination step explanation image 70E. In the determination step explanation image 70E, "prediction result check" is shown as the determination step, and an explanation of the determination content of "check prediction of machine learning model" is shown as the corresponding explanation. In addition, in the result explanation image 70D, "positive" is shown as the result of the toxicity evaluation, and an explanation of the evaluation result that "machine learning model predicts positive" is shown.

The inquiry information 74 transmitted from the server 14 is received by the client terminal 80. The expert 82 visually recognizes the content of the inquiry notification indicated by the inquiry information 74 via a display device 84. This allows the user 18 and the expert 82 to share information regarding the evaluation result.

As described above, in the information processing system 10 according to the present first modification example, the evaluation result that can be shared with the expert 82 includes the toxicity evaluation result for each toxicity item. As a result, since the evaluation result for each toxicity item can be shared, detailed information can be shared as compared with a case where only the presence or absence of toxicity is shared.

In addition, in the information processing system 10 according to the present first modification example, the toxicity evaluation result includes the toxicity determination flow 33. Among a plurality of paths in the toxicity determination flow 33, an evaluation path is shown in a distinguishable manner from the other paths. As a result, since the toxicity determination flow 33 can be shared, detailed contents can be shared as compared with a case where only the presence or absence of toxicity is shared. In addition, in the toxicity determination flow 33, the evaluation path is displayed in a distinguishable manner, so that it is easy for the sharing partner to understand which evaluation path is followed to evaluate the presence or absence of toxicity.

In addition, in the information processing system 10 according to the present first modification example, the toxicity evaluation result includes the explanation of the determination content for each determination step included in the toxicity determination flow 33. This makes it easier to understand what kind of determination is made in each determination step of the toxicity determination flow 33.

In the present first modification example, although an example of a form in which the inquiry notification includes the toxicity evaluation result for each toxicity item, the determination flow, and the explanation of the determination step has been described, the technology of the present disclosure is not limited to this. The inquiry notification may include one or two of the toxicity evaluation result for each toxicity item, the determination flow, and the explanation of the determination step. In addition, the content described in the inquiry notification may be in a form that can be appropriately set by the user 18.

### (Second Modification Example)

In the first embodiment, although an example of a form in which one compound is selected from a plurality of compounds as evaluation targets and an inquiry is made about the selected compound has been described, the technology of the present disclosure is not limited to this. In the present second modification example, in a case where a plurality of compounds are selected, an inquiry is made about each of the plurality of compounds.

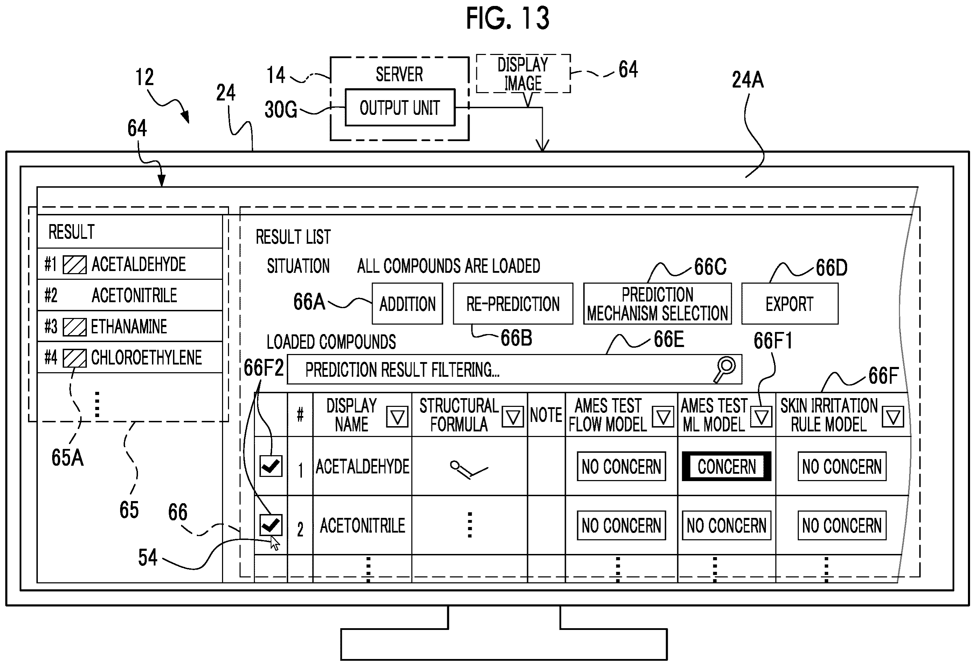

As shown in FIG. 13 as an example, a check box 66F2 is provided in the leftmost column in the result list table 66F of the result list image 66. By selecting the check box 66F2 via the pointer 54, a check mark is input in the check box 66F2, indicating that the row with the check mark has been selected. In the example shown in FIG. 13, "acetaldehyde" and "acetonitrile" are selected in the result list table 66F.

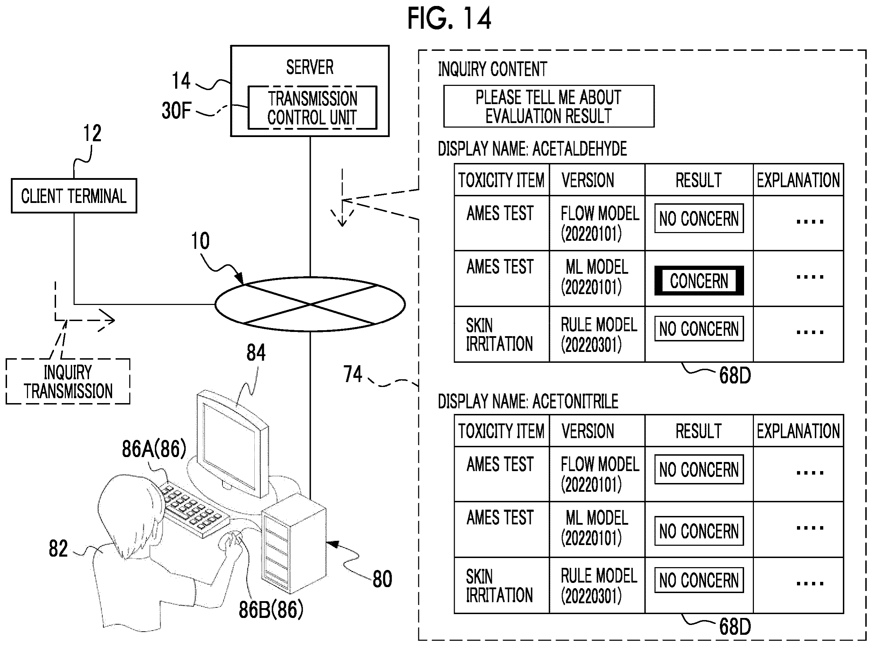

In a state where a plurality of compounds are selected in the result list table 66F, as shown in FIG. 14 as an example, by clicking the transmission key 68B via the pointer 54, the inquiry information 74, which is information indicating the inquiry notification, is transmitted from the client terminal 12 to the server 14. Then, in the server 14, the transmission control unit 30F of the processor 30 executes control of transmitting the inquiry information 74 to the client terminal 80. In the example shown in FIG. 14, an example is shown in which the inquiry information 74 indicating the inquiry notification regarding the evaluation result of acetaldehyde and the evaluation result of the acetonitrile is transmitted. Here, the inquiry information 74 includes the result overview table 68D showing the evaluation result for each toxicity item of each compound.

The inquiry information 74 transmitted from the server 14 is received by the client terminal 80. The expert 82 visually recognizes the content of the inquiry notification indicated by the inquiry information 74 via a display device 84. This allows the user 18 and the expert 82 to share information regarding evaluation results of a plurality compounds.

As described above, in the information processing system 10 according to the present second modification example, in a case where a plurality of compounds are selected in the result list table 66F of the result list image 66, the information that can be shared with the expert 82 includes the evaluation results of the plurality of compounds. This allows information sharing for the plurality of compounds collectively, thereby reducing the effort required for the user 18 to perform operations as compared with a case of sharing information for each compound one by one.

### [Second Embodiment]

In the first embodiment, although an example of a form in which the toxicity evaluation processing and the image generation processing are performed in the server 14 has been described, the technology of the present disclosure is not limited to this. In the present second embodiment, the toxicity evaluation processing is executed in the server 14, and the image generation processing is executed in the client terminal 12. In the present embodiment, the client terminal 12 is an example of an "information processing apparatus" according to the technology of the present disclosure.

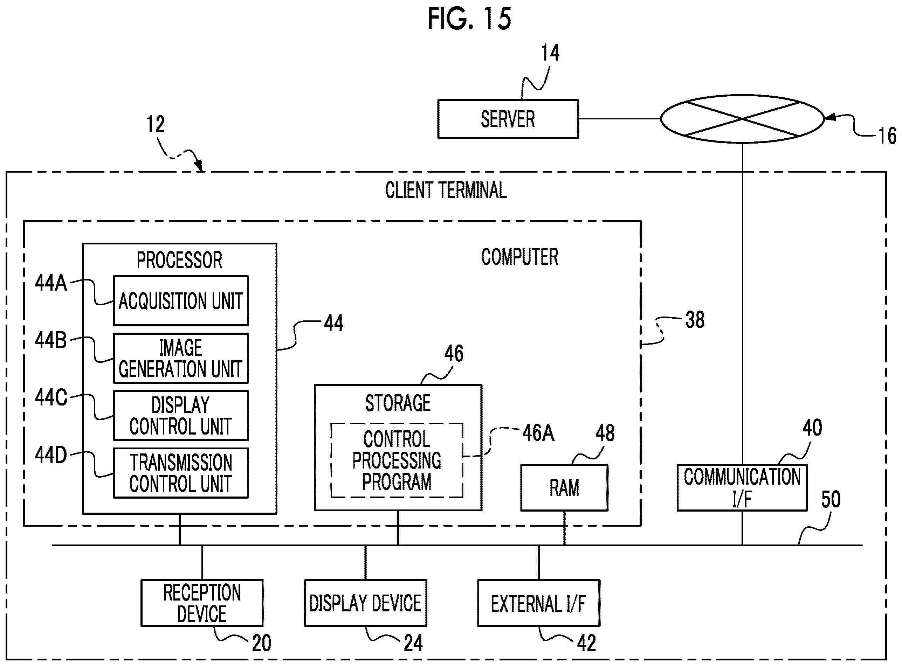

As shown in FIG. 15 as an example, the client terminal 12 comprises a computer 38, the reception device 20, the display device 24, a communication I/F 40, an external I/F 42, and a bus 50. The computer 38 comprises a processor 44, a storage 46, and a RAM 48. The processor 44, the storage 46, the RAM 48, the reception device 20, the display device 24, the communication I/F 40, and the external I/F 42 are connected to the bus 50. In the present embodiment, the processor 44 is an example of a "processor" according to the technology of the present disclosure.

In addition, since a hardware configuration (that is, the processor 44, the storage 46, and the RAM 48) of the computer 38 is basically the same as the hardware configuration of the computer 26, a description of the hardware configuration of the computer 38 will be omitted here.

The communication I/F 40 is connected to the network 16. The communication I/F 40 is responsible for transmitting and receiving information to and from an external communication device (for example, the server 14) via the network 16. For example, the communication I/F 40 transmits information in response to a request from the processor 44 to the external communication device via the network 16. In addition, the communication I/F 40 receives the information transmitted from the external communication device, and outputs the received information to the processor 44 via the bus 50.

The external I/F 42 is responsible for transmitting and receiving various types of information to and from an external device (not shown) present outside the client terminal 12. The external device may be, for example, at least one of a smart device, a personal computer, a server, a universal serial bus (USB) memory, a memory card, or a printer. An example of the external I/F 42 is a USB interface. The external device is connected directly or indirectly to the USB interface.

A control processing program 46A is stored in the storage 46. The control processing program 46A is a program for executing image generation and display control. The processor 30 executes image generation processing by reading out the control processing program 46A from the storage 46 and executing the read-out control processing program 46A on the RAM 48. The image generation processing is realized by the processor 44 operating as an acquisition unit 44A, an image generation unit 44B, a display control unit 44C, and a transmission control unit 44D. In the present embodiment, the computer 38 is an example of a "computer" according to the technology of the present disclosure, and the control processing program 46A is an example of a "program" according to the technology of the present disclosure.

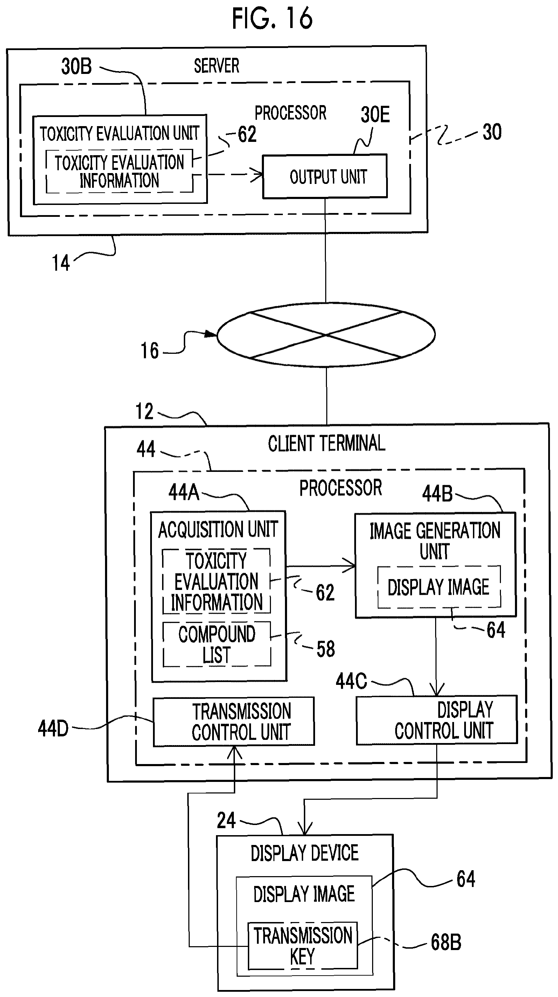

As shown in FIG. 16 as an example, in the processor 30 of the server 14, the toxicity evaluation unit 30B outputs the toxicity evaluation information 62 obtained by the toxicity evaluation processing to the output unit 30E. The output unit 30E outputs the toxicity evaluation information 62 to the client terminal 12 via the network 16.

In the processor 44 of the client terminal 12, the acquisition unit 44A acquires the toxicity evaluation information 62 via the network 16. Then, the acquisition unit 44A outputs the compound list 58 and the toxicity evaluation information 62 to the image generation unit 44B. The image generation unit 44B generates a display image 64 based on the compound list 58 and the toxicity evaluation information 62. Then, the image generation unit 44B outputs the generated display image 64 to the display control unit 44C. The display control unit 44C performs graphical user interface (GUI) control for displaying the display image 64, thereby causing the display device 24 to display the display image 64.

The display image 64 includes the transmission key 68B. In a case where the transmission key 68B is operated by the user 18, the transmission control unit 44D executes control of transmitting the inquiry information 74 to a destination (for example, the client terminal 80) designated in advance. In this case, the transmission control unit 30F may transmit the inquiry information 74 via the server 14, or may directly communicate with the client terminal 80 to transmit the inquiry information 74.

### [Third Embodiment]

In the first embodiment, although an example of a form in which the toxicity evaluation processing and the image generation processing are performed in the server 14 has been described, the technology of the present disclosure is not limited to this. In the present third embodiment, the toxicity evaluation processing and the image generation processing are performed in the client terminal 12. In the present embodiment, the client terminal 12 is an example of an "information processing apparatus" according to the technology of the present disclosure.

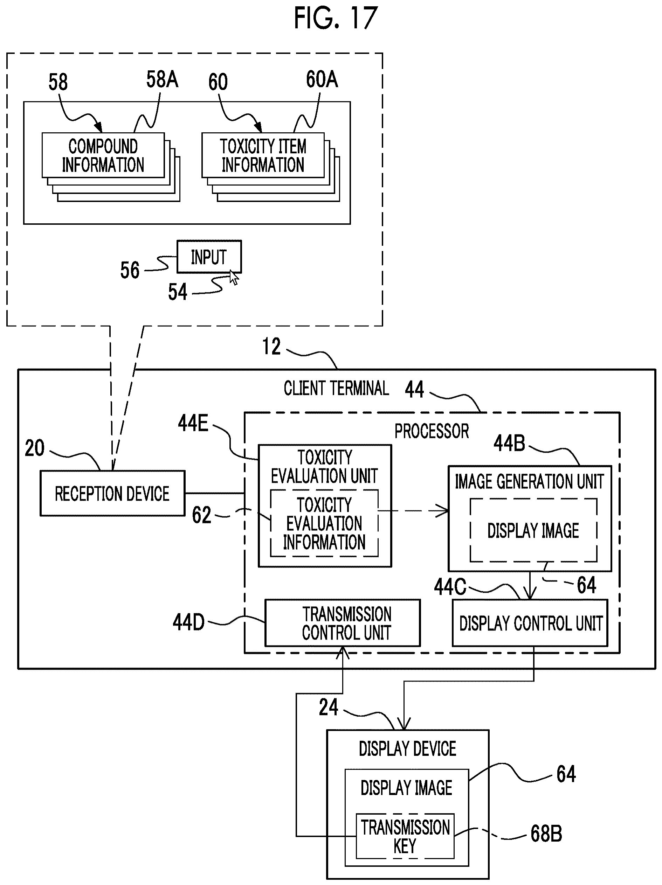

As shown in FIG. 17 as an example, the compound list 58 and the toxicity item list 60 are received in the client terminal 12 via the reception device 20. In the processor 44 of the client terminal 12, the toxicity evaluation unit 44E performs the toxicity evaluation indicated by the toxicity item list 60 on the compound group indicated by the compound list 58. Then, the image generation unit 44B generates a display image 64 based on the toxicity evaluation information 62 and the compound list 58. The display control unit 44C causes the display device 24 to display the display image 64.

The display image 64 includes the transmission key 68B. In a case where the transmission key 68B is operated by the user 18, the transmission control unit 44D executes control of transmitting the inquiry information 74 to a destination (for example, a terminal used by the expert 82) designated in advance. In this case, the transmission control unit 30F may activate email software or chat software installed on the client terminal 12 to transmit the inquiry information 74, or may directly communicate with a terminal used by the expert 82 to transmit the inquiry information 74.

In the above-described embodiments, although an example of a form in which the inquiry notification indicated by the inquiry information 74 includes the text and/or the image indicating the toxicity evaluation result has been described, the technology of the present disclosure is not limited to this. For example, the inquiry notification may include an identifier (for example, a two-dimensional matrix image) indicating the toxicity evaluation result, may include a link to display the display image 64, or may include the display image 64 itself.

In addition, in the above-described embodiments, although an example of a form in which the toxicity evaluation information 62 is obtained by executing the toxicity evaluation processing has been described, the technology of the present disclosure is not limited to this. For example, the server 14 or the client terminal 12 may receive already obtained toxicity evaluation information 62 (for example, toxicity evaluation information 62 obtained as a result of processing by an external device or toxicity evaluation information 62 obtained in the past), and perform the image generation processing.

In addition, in the above-described embodiments, although an example of a form in which the evaluation processing program 32A and the generation processing program 32B are stored in the storage 32 has been described, the technology of the present disclosure is not limited to this. For example, the evaluation processing program 32A and the generation processing program 32B may be stored in a portable storage medium such as an SSD or a USB memory. The storage medium is a non-transitory computer-readable storage medium. The evaluation processing program 32A and the generation processing program 32B stored in the storage medium are installed in the computer 26. The processor 30 executes control processing of the server 14 in accordance with the evaluation processing program 32A and the generation processing program 32B. Similarly, the control processing program 46A may be stored in a storage medium instead of the storage 46.

In the above-described embodiments, the computers 26 and 38 are exemplified, but the technology of the present disclosure is not limited to this, and a device including an application specific integrated circuit (ASIC), a field programmable gate array (FPGA), and/or a programmable logic device (PLD) may be applied instead of the computers 26 and 38. In addition, instead of the computers 26 and 38, a hardware configuration and a software configuration may be used in combination.

The following various processors can be used as hardware resources for executing the various kinds of processing described in the above-described embodiments. Examples of the processor include a CPU that is a general-purpose processor that functions as a hardware resource for executing the software, that is, the program to execute the toxicity evaluation processing and/or the image generation processing (hereinafter, also simply referred to as "various kinds of processing"). Examples of the processor also include a dedicated electronic circuit that is a processor whose dedicated circuit configuration is specially designed to execute specific processing, such as an FPGA, a PLD, or an ASIC. Any processor includes a memory built therein or connected thereto, and any processor uses the memory to execute various kinds of processing.

The hardware resource for executing the various kinds of processing may be configured by one of the various processors or by a combination of two or more processors of the same type or different types (for example, a combination of a plurality of FPGAs or a combination of a processor and an FPGA). Further, the hardware resource for executing the various kinds of processing may be one processor.

A first example of the configuration in which the hardware resource is configured by one processor is an aspect in which one processor is configured by a combination of one or more processors and software, and this processor functions as the hardware resource for executing the various kinds of processing. Second, as represented by system-on-a-chip (SoC), there is a form in which a processor that realizes the functions of the entire system including a plurality of hardware resources for executing the various kinds of processing with a single integrated circuit (IC) chip. As described above, the various kinds of processing are implemented by using one or more of the various processors as the hardware resource.

Further, specifically, an electronic circuit in which circuit elements, such as semiconductor elements, are combined can be used as the hardware structure of these various processors. The various kinds of processing are merely examples. Accordingly, it is possible to delete an unnecessary step, add a new step, or change a processing order without departing from the gist of the present disclosure.

The content of the above description and the content of the drawings are detailed explanations of the parts relating to the technology of the present disclosure, and are merely examples of the technology of the present disclosure. For example, description related to the above configurations, functions, actions, and effects is description related to an example of configurations, functions, actions, and effects of the parts relating to the technology of the present disclosure. Thus, it is needless to say that unnecessary portions may be deleted, new elements may be added, or replacement may be made to the content of the above description and the content of the drawings without departing from the gist of the technology of the present disclosure. In order to avoid complication and easily understand the parts relating to the technology of the present disclosure, in the content of the above description and the content of the drawings, the description regarding common general technical knowledge which is not necessarily particularly described in terms of embodying the technology of the present disclosure is omitted.

In the present specification, "A and/or B" is synonymous with "at least one of A or B". That is, "A and/or B" may refer to A alone, B alone, or a combination of A and B. In addition, in the present specification, in a case in which three or more matters are expressed with the connection of "and/or", the same concept as "A and/or B" is applied.

All documents, patent applications, and technical standards mentioned in the present specification are incorporated herein by reference to the same extent as in a case in which each document, each patent application, and each technical standard are specifically and individually described by being incorporated by reference.

The disclosure of JP2023-059424 filed on March 31, 2023 is incorporated herein by reference in its entirety.

In regard to the embodiments described above, the following appendices will be further disclosed.

### <Appendix 1>

An information processing apparatus comprising:
a processor,
in which the processor is configured to
   output, to a display unit, an inquiry image in which an evaluation result of a toxicity evaluation of a compound is capable of being displayed, the inquiry image including an operation unit that receives an operation of transmitting an inquiry notification regarding the evaluation result to a destination designated in advance, and
   in a case where the operation unit is operated, execute control of transmitting, to the destination, the inquiry notification including information that is shareable with the destination and that includes at least a part of the evaluation result.

### <Appendix 2>

The information processing apparatus according to Appendix 1,
in which the inquiry image is displayed by transitioning from a list image showing a result of performing the toxicity evaluation on each of compounds included in a plurality of compound groups as toxicity evaluation targets, in a case where at least one of the compounds is selected from the list image.

### <Appendix 3>

The information processing apparatus according to Appendix 2,
in which the list image is capable of being sorted and/or filtered out according to a condition designated by a user.

### <Appendix 4>

The information processing apparatus according to any one of Appendices 1 to 3,
in which the evaluation result includes a toxicity evaluation result for each toxicity item.

### <Appendix 5>

The information processing apparatus according to Appendix 4,
in which the toxicity evaluation result includes a determination flow indicating a toxicity determination procedure, which is used in the toxicity evaluation, has at least one determination step, and has a path that branches according to a determination result of the determination step, and in which, among a plurality of paths, a path that leads to the toxicity evaluation result of the compound is shown in a distinguishable manner from the other paths.

### <Appendix 6>

The information processing apparatus according to Appendix 5,
in which the toxicity evaluation result includes an explanation of a determination content for each determination step included in the determination flow.

### <Appendix 7>

The information processing apparatus according to any one of Appendices 1 to 6,
in which the inquiry image includes an input region in which an inquiry content included in the inquiry notification is capable of being input.

### <Appendix 8>

The information processing apparatus according to Appendix 7,
in which the input region is a text input region in which a text indicating the inquiry content is capable of being input.

### <Appendix 9>

The information processing apparatus according to Appendix 2 and any one of Appendices 3 to 8 citing Appendix 2,
in which, in a case where a plurality of compounds are selected in the list image, the information that is shareable with the destination includes at least a part of the evaluation result of each of the plurality of compounds.

## Claims

1. An information processing apparatus comprising:
a processor,
wherein the processor is configured to
output, to a display unit, an inquiry image in which an evaluation result of a toxicity evaluation of a compound is capable of being displayed, the inquiry image including an operation unit that receives an operation of transmitting an inquiry notification regarding the evaluation result to a destination designated in advance, and
in a case where the operation unit is operated, execute control of transmitting, to the destination, the inquiry notification including information that is shareable with the destination and that includes at least a part of the evaluation result.

2. The information processing apparatus according to claim 1,
wherein the inquiry image is displayed by transitioning from a list image showing a result of performing the toxicity evaluation on each of compounds included in a plurality of compound groups as toxicity evaluation targets, in a case where at least one of the compounds is selected from the list image.

3. The information processing apparatus according to claim 2,
wherein the list image is capable of being sorted and/or filtered out according to a condition designated by a user.

4. The information processing apparatus according to claim 1,
wherein the evaluation result includes a toxicity evaluation result for each toxicity item.

5. The information processing apparatus according to claim 4,
wherein the toxicity evaluation result includes a determination flow indicating a toxicity determination procedure, which is used in the toxicity evaluation, has at least one determination step, and has a path that branches according to a determination result of the determination step, and in which, among a plurality of paths, a path that leads to the toxicity evaluation result of the compound is shown in a distinguishable manner from the other paths.

6. The information processing apparatus according to claim 5,
wherein the toxicity evaluation result includes an explanation of a determination content for each determination step included in the determination flow.

7. The information processing apparatus according to claim 1,
wherein the inquiry image includes an input region in which an inquiry content included in the inquiry notification is capable of being input.

8. The information processing apparatus according to claim 7,
wherein the input region is a text input region in which a text indicating the inquiry content is capable of being input.

9. The information processing apparatus according to claim 2,
wherein, in a case where a plurality of compounds are selected in the list image, the information that is shareable with the destination includes at least a part of the evaluation result of each of the plurality of compounds.

10. An information processing method comprising:
outputting, to a display unit, an inquiry image in which an evaluation result of a toxicity evaluation of a compound is capable of being displayed, the inquiry image including an operation unit that performs an operation of transmitting an inquiry notification regarding the evaluation result to a destination designated in advance; and
executing, in a case where the operation unit is operated, control of transmitting, to the destination, the inquiry notification including information that is shareable with the destination and that includes at least a part of the evaluation result.

11. A program for causing a computer to execute a process comprising:
outputting, to a display unit, an inquiry image in which an evaluation result of a toxicity evaluation of a compound is capable of being displayed, the inquiry image including an operation unit that performs an operation of transmitting an inquiry notification regarding the evaluation result to a destination designated in advance; and
executing, in a case where the operation unit is operated, control of transmitting, to the destination, the inquiry notification including information that is shareable with the destination and that includes at least a part of the evaluation result.
